# EUROPÄISCHE PATENTSCHRIFT

(11) **EP 2 339 980 B1**
(45) Veröffentlichungstag und Bekanntmachung des Hinweises auf die Patenterteilung: **16.07.2014**
(21) Anmeldenummer: 09772076.7
(22) Anmeldetag: 10.06.2009
(51) Int. Cl.: A61B 17/00, A61B 18/02

(54) **TEMPERATURREGELUNG FÜR EINE KRYOSONDE, KRYOCHIRURGISCHES GERÄT MIT TEMPERATURREGLER UND VERFAHREN ZUM REGELN DER TEMPERATUR EINER KRYOSONDE**
TEMPERATURE CONTROLLER FOR A CRYOPROBE, CRYOSURGICAL DEVICE HAVING A TEMPERATURE CONTROLLER, AND METHOD FOR REGULATING THE TEMPERATURE OF A CRYOPROBE
RÉGULATION THERMIQUE POUR UNE CRYOSONDE, APPAREIL CRYOCHIRURGICAL AVEC RÉGULATEUR THERMIQUE ET PROCÉDÉ DE RÉGULATION DE LA TEMPÉRATURE D'UNE CRYOSONDE

(30) Priorität: 02.07.2008 DE 102008031298; 03.09.2008 DE 102008045563
(43) Veröffentlichungstag der Anmeldung: 06.07.2011
(73) Patentinhaber: ERBE Elektromedizin GmbH, 72072 Tübingen (DE)
(72) Erfinder: GEISELHART, Franz, 72770 Reutlingen (DE)
(74) Vertreter: Bohnenberger, Johannes
(86) Internationale Anmeldenummer: PCT/EP2009/004209
(87) Internationale Veröffentlichungsnummer: WO 2010/000376

(56) Entgegenhaltungen:
- EP-A- 1 398 002
- WO-A-00/42932

## Beschreibung

Die Erfindung betrifft einen Temperaturregler zur Regelung der Temperatur einer Krposonde gemäß Anspruch 1, ein kryochirurgisches Gerät mit einem derartigen Temperaturregler gemäß Anspruch 9 und ein Verfahren zum Regeln der Temperatur einer Kryosonde gemäß Anspruch 11.

Kryochirurgische Geräte sind bekannt. Sie umfassen ein kryochirurgisches Instrument, eine Fluidquelle und eine Steuerung zum Einstellen der Kühlleistung, die an dem kryochirurgischen Instrument bereitgestellt wird.

Für die Bereitstellung der Kühlleistung verwendet man entweder eine Kaltdampfanlage oder den Joule-Thomson Effekt. Der Joule-Thomson Effekt tritt auf, wenn ein reales Gas oder Gasgemisch durch Drosselung (Druckänderung) eine Temperaturänderung erfährt. Drosselt man ein Gas, etwa indem man eine Drossel einsetzt oder ein anderes Hindernis einbaut, so expandiert es. Das heißt, das vom Gas eingenommene Volumen hinter dem Hindernis nimmt zu. Dabei erhöht sich der mittlere Teilchenabstand, wodurch die Temperatur des Gases sinkt.

Die Kaltdampfanlage nutzt die Eigenschaft von Stoffen, bei unterschiedlichen Drücken unterschiedliche Siede- bzw. Kondensationstemperaturen zu haben. Die verwendeten und bevorzugten Stoffe nennt man Kältemittel. Der Arbeitsbereich einer Kaltdampfanlage ist begrenzt durch die erreichbaren Siede- bzw. Kondensationstemperaturen des Kältemittels. Üblicherweise wird ein verflüssigtes Kältemittel unter hohem Druck in eine Verdunstungskammer eingespritzt, in der ein wesentlich niedrigerer Druck vorliegt. Der Siedepunkt des entsprechenden Kältemittels hängt zum einen von dem verwendeten Kältemittel ab, zum anderen von dem in der Verdunstungskammer vorherrschenden Druck. Soweit sich also in der Verdunstungskammer ein Siedepunkt ergibt, der oberhalb der vorherrschenden Temperatur in der Verdunstungskammer liegt, verdampft das Kältemittel zumindest teilweise und entzieht dem System Wärmeenergie. Das verdampfte Kältemittel wird durch einen Ablauf wieder abgefühlt und entweder erneut kondensiert oder entsorgt.

Für die effektive und vorteilhafte Verwendung von kryochirurgischen Instrumenten ist es notwendig, die Temperatur der Sonde oder eines Teils dieser auf einen vorgegebenen Wert einstellen zu können und bei diesem konstant zu halten. In der Kryochirurgie lässt sich so beispielsweise bei gleichen Indikationen ein reproduzierbarer Gewebeeffekt erzielen. Ebenso ist es wünschenswert, für unterschiedliche Indikationen die Temperatur der Sondenspitze variieren zu können, um den jeweils optimalen Gewebeeffekt zu erzielen.

Um eine exakte Regelung der Kühlleistung und der an der Sondenspitze vorherrschenden Temperatur bewerkstelligen zu können, umfassen moderne kryochirurgische Geräte Temperaturregler.

Für die Reglung ist es notwendig, die Temperatur an der Sondenspitze zu bestimmen. Derzeit werden Miniaturmantelthermoelemente als Temperatursensoren verwendet. Diese sind relativ teuer, ihre Montage aufwändig und die Haltbarkeit aufgrund der geringen mechanischen Belastbarkeit und der Korrosionsanfälligkeit relativ gering. Für sehr dünne Sonden existieren keine Thermoelemente, die eine entsprechende Dimensionierung aufweisen. Deshalb gibt es bestimmte Sonden (z.B. Endokryosonden und Intravitrialsonden) nur ohne Temperaturregelung.

Aus der EP-A-1 398 002 ist eine Ablationssonde bekannt, bei der ein vorgekühltes Kältemittel im Distalbereich verdunstet wird, um die Sonde abzukühlen. Zur geeigneten Abkühlung der Sonde sind ein Primärkühlkreislauf und ein Sekundärkühlkreislauf vorgesehen. Beim Primärkühlkreislauf wird das Kältemittel von einer Kältemittelquelle über ein Ventil an einem Sensor vorbei in den Distalbereich der Ablationssonde geführt. Das Kältemittel kann nach der Expansion im Distalbereich abgeführt werden, wobei der im Ablauf anliegende Druck mittels

eines Sensors gemessen wird. Der Sekundärkreislauf kühlt das Kältemittel im Primärkreislauf vor. Somit ist aus der EP-A-1 398 002 eine Druckmessung im Zulauf und im Ablauf bekannt. Die Druckschrift verwendet jedoch Temperatursensoren oder Drucksensoren, um eine geeignete Temperatur im Distalbereich der Ablationssonde einzustellen. Dies ist aus den genannten Gründen problematisch.

Die WO 00/42932 A beschreibt eine weitere Kryosonde.

Häufig verwendet man kryochiturgische Geräte, deren Instrumente umschaltbare Drosseln im Rücklauf enthalten. Beispielsweise haben diese Drosseln drei einstellbare Stufen. Die Temperatur lässt sich bei einer halbwegs konstanten Fluidquelle zwischen den einzelnen einstellbaren Stufen regeln. Durch ein wiederholtes Einsetzen der Instrumente oder durch ein Ausmessen der Kühlleistung lassen sich Referenzwerte ermitteln. Die so bestimmten Referenzwerte sind relativ ungenau und schwanken abhängig von dem Einsatzgebiet. Außerdem müssen für sämtliche verwendete Instrumente neue Messungen durchgeführt werden. Dies ist sehr aufwändig.

Ausgehend von der EP-A-1 398 002 ist es Aufgabe der vorliegenden Erfindung, einen verbesserten Temperaturregler zur Regelung der Temperatur einer Kryosonde bereitzustellen. Insbesondere soll ein Temperaturregler aufgezeigt werden, der in sämtlichen Kryosonden unabhängig von deren Bauart eingesetzt werden kann. Des Weiteren soll ein entsprechendes kryochirurgiscles Gerät und Verfahren zum Regeln der Temperatur einer Kryosonde angegeben werden.

Diese Aufgabe wird erfindungsgemäß durch einen Temperaturregler gemäß Anspruch 1, ein kryochirurgisches Gerät gemäß Anspruch 9 und ein Verfahren gemäß Anspruch 11 gelöst.

Ein wesentlicher Gedanke der vorliegenden Erfindung besteht also darin, den Druck in dem Verdunstungsbereich derart einzustellen, dass sich für das verwendete Kältemittel ein Siedepunkt ergibt, der im Wesentlichen gleich oder nahe der gewünschten Zieltemperatur in der Kryosonde ist. Soweit die Kryosonde diese Zieltemperatur bereits erreicht hat, kommt es also zu keiner Verdunstung mehr und es wird keine Kühlleistung in der Kryosonde erbracht. Liegt die Temperatur in dem Verdunstungsbereich oberhalb des eingestellten Siedepunkts, so kommt es zu einer Verdunstung, die dem Verdunstungsbereich und somit der Kryosonde Wärmeenergie entzieht. Somit kann auf die Bestimmung der vorliegenden Temperatur in dem Verdunstungsbereich oder an der Kryosonde verzichtet werden, da das System bei vorher eingestelltem Siedepunkt selbstregelnd ist.

Während bei Kältemitteln im engeren Sinn Wärme durch Verdampfung bei niedrigem Druck und niedriger Temperatur aufgenommen wird, geschieht dies in einer Kältemischung chemisch durch eine Mischungs- und Lösungsreaktion. Die Regeneration erfolgt daher bei Kältemischungen durch Entmischung. In dieser Anmeldung soll der Begriff Kältemittel in seiner allgemeinen Bedeutung verstanden werden, also auch Kältemischungen umfassen.

Die Druckeinstelleinrichtung kann mindestens einen ersten Druckfühler zum Messen des zweiten Drucks und mindestens ein einstellbares Ventil umfassen, die über eine Regeleinrichtung zum Regeln des zweiten Drucks verbunden sind. Da der Siedepunkt unmittelbar von dem vorliegenden Druck abhängt, lässt sich die Temperatur der Kryosonde durch die Vorgabe eines bestimmten Druckes in dem Verdunstungsbereich einstellen. Mittels eines Ventils und eines entsprechenden Druckfühlers kann also die Temperatur geregelt werden. Die Regeleinrichtung ist also derart ausgebildet, dass sie je nach Temperaturvorgabe einen konstanten Druck in dem Verdunstungsbereich einstellt. Die Regelung der Temperatur über ein Druckfühlerventil hat den weiteren Vorteil, dass diese Elemente nicht zwingend innerhalb der Kryosonde angeordnet sein müssen. Es ist denkbar, den Druckfühler und das Ventil am Ablauf der Kryosonde vorzusehen. Des Weiteren können diese Elemente beispielsweise innerhalb eines Handgriffs der Kryosonde angeordnet werden. Im Vergleich zu der Anordnung mit Temperatursensor ist es nicht notwendig, dass die Elemente unmittelbar in oder an dem Verdunstungsbereich, der üblicherweise räumlich stark begrenzt ist, angeordnet sind.

Der Temperaturregler kann mindestens einen Druckminderer zum Regeln des zweiten Drucks umfassen.

Der Temperaturregler kann eine Strömungsmesseinrichtung zum Bestimmen einer Strömung, die bei einem Abfluss des Kältemittels aus dem Verdunstungsbereich auftritt, und eine Kompensationseinrichtung zum Einbeziehen eines dem Abfluss entgegenstehenden Strömungswiderstands beim Messen und/oder Einstellen des zweiten Drucks umfassen. Entscheidet man sich für einen Druckfühler, der von dem Verdunstungsbereich entfernt angeordnet ist, so ist es hilfreich, einen auftretenden Strömungswiderstand zu berücksichtigen, der zwischen dem Messbereich und dem Verdunstungsbereich auftritt. Bei tiefen Temperaturen, wenn das zurückfließende Gas einen kleinen Druck und somit ein großes spezifisches Volumen aufweist, ist der Strömungswiderstand hoch und deshalb an dieser Stelle die Korrektur besonders wichtig. Hinzu kommt, dass in diesem Temperaturbereich die Siedelinie besonders steil verläuft und somit ein kleiner Fehler bei der Messung des Drucks einen großen Fehler bezüglich der Temperatur zur Folge hat.

Die Strömungsmesseinrichtung kann zum Bestimmen der Strömung einen zweiten Druckfühler zum Erfassen des ersten Drucks umfassen. Es ist also möglich, anhand der Druckdifferenz im Zulauf und im Ablauf auf den Strömungswiderstand zu schließen. Alternativ kann der Gasdurchfluss an geeigneter Stelle Aufschluss über den zu berücksichtigenden Strömungswiderstand geben.

Die Kompensationseinrichtung kann eine Dateneingabevorrichtung zur Eingabe von Strömungswiderstandsdaten der Kryosonde umfassen. Da der Strömungswiderstand abhängig von der Ausgestaltung der jeweiligen Kryosonde ist, insbesondere vom Querschnitt des Rücklaufs, kann der Strömungswiderstand vorab bestimmt werden und über eine Dateneingabevorrichtung manuell oder automatisch eingegeben werden.

Beispielsweise kann die Dateneingabevorrichtung einen Datenspeicher umfassen, der Strömungswiderstandsdaten einer Vielzahl von Kryosonden enthält. Somit wird die Bestimmung des Strömungswiderstands wesentlich vereinfacht.

Die Dateneingabevorrichtung kann eine Geräteidentifikationseinrichtung zur Identifikation einer angeschlossenen Kryosonde umfassen. Somit ist es denkbar, dass der Temperaturregler eine angeschlossene Kryosonde automatisch identifiziert und entsprechende Strömungswiderstandsdaten aus einem Datenspeicher ausliest. Diese Strömungswiderstandsdaten können verwendet werden um bei der Messung und/oder Einstellung des zweiten Drucks berücksichtigt zu werden.

Die Aufgabe wird des Weiteren durch ein kryochirurgisches Gerät, umfassend eine Kryosonde mit einem Sondenschaft und einem Temperaturregler, wie vorab beschrieben, gelöst. Die Vorteile ergeben sich in ähnlicher Weise, wie das bereits dargestellt wurde. Die Kryosonde kann mindestens einen ersten Druckfühler umfassen, der zum Erfassen des zweiten Drucks am oder nahe dem proximalen Ende des Sondenschafts angeordnet ist. Somit kann die besagte Druckeinstelleinrichtung dort angeordnet werden, wo ausreichend Platz für die entsprechenden Elemente, z.B. Ventile oder Druckfühler, vorhanden ist.

Des Weiteren wird die Aufgabe durch ein Verfahren zum Regeln der Temperatur einer Kryosonde gelöst, wobei die Kryosonde einen Verdampfungsbereich zum Verdampfen eines Kältemittels aufweist und das Verfahren folgende Schritte umfasst:
- Bestimmen mindestens eines Drucks des Kältemittels im Verdampfungsbereich;
- Einstellung des Drucks auf einen vorbestimmten Wert, um die Temperatur der Kryosonde zu regeln;

Ein zentraler Gedanke besteht also auch bei dem Verfahren darin, die Temperaturregelung anhand der Einstellung eines bestimmten Drucks in dem Verdampfungsbereich zu gewährleisten. Somit erfolgt eine indirekte Temperaturregelung über den Druck.

Das Bestimmen des mindestens einen Drucks kann mittels eines Druckfühlers in der Kryosonde an deren proximalen Ende oder bei der Ableitung des Kältemittels aus der Kryosonde erfolgen. Bei der Bestimmung des Drucks ist man also nicht daran gebunden, diesen unmittelbar in dem Verdampfungsbereich zu messen.

Auch bei dem Verfahren gilt es, den Strömungswiderstand zu berücksichtigen.

Nachfolgend wird die Erfindung anhand einiger Ausführungsbeispiele beschrieben, die mittels einer Abbildung näher erläutert werden. Hierbei zeigt:

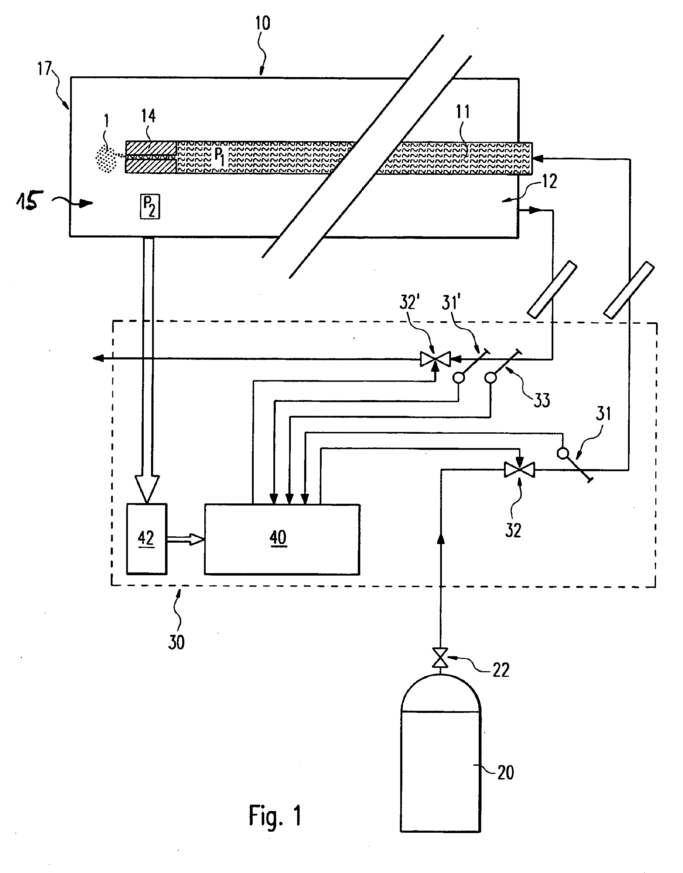

- Fig. 1 eine schematische Darstellung einer Kryosonde mit einem Temperaturregler.

Das erfindungsgemäße kryochirurgische Gerät umfasst eine Fluidquelle 20 zum Bereitstellen eines Kältemittels 1 mit einem vorbestimmten Druck. Das Kältemittel 1 wird über einen Temperaturregler 30 einer Kryosonde 10 zugeführt. Im Inneren der Kryosonde 10 verläuft ein Zulauf 11, der das Kältemittel 1 in einen Bereich nahe der Sondenspitze 17 leitet. Innerhalb des Zulaufs 11 hat das Kältemittel einen ersten Druck P₁, der derart hoch ist, dass der sich ergebende kältemittelspezifische Siedepunkt deutlich niedriger ist, als die Temperatur, die in der Kryosonde 10 vorliegt. Das Kältemittel 1 behält also seinen flüssigen Aggregatszustand. Über eine Düse 14 am distalen Ende des Zulaufs 11 wird das Kältemittel 1 in einen innerhalb der Kryosonde 10 angeordneten Verdunstungsbereich 15 abgegeben. Der hier vorliegende zweite Druck P₂ ist deutlich niedriger als der erste Druck P₁. Somit ergibt sich kältemittelspezifisch ein niedrigerer Siedepunkt. Das Kältemittel 1 verdunstet abhängig von der in der Kryosonde 10 vorliegenden Temperatur und entzieht dieser Wärmeenergie.

Alternativ kann das Kältemittel 1 in Gasform bei maximalem Betriebsdruck (Gasentnahme an der Siedelinie) derart zugeführt werden, dass das Gas bei der Abkühlung durch den Joule-Thomson Effekt an der Düse 14 sich in ein Zweiphasengemisch wandelt. Der flüssige Anteil siedet an der Innenoberfläche des Sondenkopfs bzw. der Sondenspitze 17 im Verdunstungsbereich 15.

Das Kältemittel 1 oder Gas wird dann über einen Ablauf 12 innerhalb der Kryosonde 10 in den Temperaturregler 30 zurückgeführt und von dort entsorgt oder wieder aufbereitet. Hierfür steht der Ablauf 12 in fluider Verbindung mit dem Verdunstungsbereich 15 und dem Temperaturregler 30.

Durch die Regelung des zweiten Drucks P₂ kann die Siedetemperatur entsprechend einem vorgegebenen Wert eingestellt werden. Es ist also möglich, eine direkte Korrelation zwischen dem zweiten Druck P₂ und der Kühlleistung der Kryosonde 10 herzustellen.

Um die Temperatur der Kryosonde 10 effektiv zu regeln, umfasst der Temperaturregler 30 eine Steuerung 40 sowie einen Zulaufdruckfühler 31, einen Ablaufdruckfühler 31', ein Zulaufproportionalventil 32, ein Ablaufproportionalventil 32' und einen Durchflusssensor 33. Der Zulaufdruckfühler 31 gibt Signale aus, die es der Steuerung 40 ermöglichen, auf den ersten Druck P₁ im Zulauf 11 zu schließen. Hierfür ist der Zulaufdruckfühler 31 an dem Zulauf 11 angeordnet. Der Ablaufdruckfühler 31' ist dementsprechend an dem Ablauf 12 angeordnet und gibt Signale aus, mittels derer die Steuerung 40 den zweiten Druck P₂ innerhalb des Verdunstungsbereichs 15 ermitteln kann. Um den Druckabfall, der beim Ausströmen des Kältemittels 1 aus dem Verdunstungsbereich 15 über den Ablauf 12 auftritt, berücksichtigen zu können, befindet sich an dem Ablauf 12 ebenfalls ein Druckflusssensor 33 mittels dessen die Steuerung 40 auf den vorliegenden Strömungswiderstand schließen kann. Somit kann die Steuerung 40 den mit dem Ablaufdruckfühler 31 gemessenen Druck unter Berücksichtigung des Strömungswiderstands korrigieren und einen entsprechend bereinigten Druck P₂ ermitteln.

Diese Korrektur ist besonders wichtig, wenn die Kryosonde 10 bei tiefen Temperaturen betrieben wird. Das zurückfließende Gas bzw. das Kältemittel 1 weist dann einen sehr niedrigen Druck P₂ und somit ein großes spezifisches Volumen auf, wodurch ein hoher Strömungswiderstand auftritt. Hinzu kommt, dass in diesem Temperaturbereich die Siedelinie von üblichen Kältemitteln 1 besonders steil verläuft und somit ein kleiner Fehler bei der Berechnung des zweiten Drucks P₂ eine starke Abweichung bei der Einstellung der Temperatur in der Kryosonde 10 zur Folge hat.

Es ist ein Vorteil der vorliegenden Erfindung, dass die Sensoren und Fühler zur Bestimmung des ersten Drucks P₁ und/oder des zweiten Drucks P₂ nicht in oder nahe dem Verdunstungsbereich 15 angeordnet werden müssen.

Diese Sensoren oder Fühler können in den Ablauf 12 bzw. Zulauf 11 oder an dessen proximalen Ende angebracht sein. Somit ist eine Konstruktion von Kryosonden 10 mit deutlich geringerem Durchmesser denkbar.

In einem zweiten Ausführungsbeispiel kann der Strömungswiderstand alleine anhand des Zulaufdruckfühlers 31 und des Ablaufdruckfühlers 31' ermittelt werden. Hierfür ist es notwendig, gerätespezifische Daten über die an den Temperaturregler 30 angeschlossene Kryosonde 10 bereitzustellen. Diese Daten umfassen beispielsweise den Querschnitt der Düse 14. Alternativ können im Labor Messungen des Strömungswiderstands abhängig von dem ersten und/oder zweiten Druck P₁, P₂ vorgenommen und tabellarisch erfasst werden. Um die gerätespezifischen Daten einer bestimmten Kryosonde 10 zuordnen zu können, umfasst ein Ausführungsbeispiel eine Dateneingabevorrichtung. Diese Dateneingabevorrichtung hat einen Speicher zum Speichern der gerätespezifischen Daten und eine Geräteerkennung, die es ermöglicht, einen spezifischen Kryosondentyp 10 auszuwählen. Sobald eine bestimmte Kryosonde 10 ausgewählt ist, berechnet die Steuerung 40 den gerätespezifischen Strömungswiderstand abhängig von den Druckverhältnissen und den gespeicherten geräte- bzw. instrumentenspezifischen Daten.

Alternativ kann die Dateneingabevorrichtung 42 eine automatische Erfassung des angeschlossenen Gerätetyps vornehmen.

Diese automatische Erkennung kann beispielsweise durch das Auslesen einer Seriennummer, die in einem an der Kryosonde 10 angebrachten RFID-Tag gespeichert ist, erfolgen.

Dem Fachmann sollten zahlreiche weitere Verfahren bekannt sein, wie eine entsprechende Identifizierung der angeschlossenen Kryosonde 10 ermöglicht werden kann. Hierunter fallen unter anderem die Ermittlung von verschiedenen Kenngrößen in einer einer Operation vorgeschalteten Testphase der Kryosonde 10, das Auslesen eines Bluetooth-Tags oder das Einscannen eines Barcodes.

Um eine möglichst fehlertolerante Ermittlung des zweiten Drucks P₂ vornehmen zu können, ist es denkbar, den Strömungswiderstand gemäß dem Verfahren in dem ersten Ausführungsbeispiel und dem in dem zweiten Ausführungsbeispiel zu ermitteln. Alternativ kann lediglich eines der beiden Verfahren angewandt werden.

Es sollte für den Fachmann offensichtlich sein, dass sich das erfindungsgemäße Verfahren zur Regelung der Temperatur der Kryosonde 10 auch durchführen lässt, wenn lediglich ein Ablaufdruckfühler 31' und ein Ablaufproportionalventil 32' vorhanden ist.

Die Steuerung 40 kann also nach der Ermittlung des zweiten Drucks P₂ eine Regelschleife implementieren, bei der das Ablaufproportionalventil 32', das an dem Ablauf 12 angeschlossen ist, derart eingestellt wird, dass der Druck P₂ einen vorgegebenen Wert erreicht. Dieser Wert korrespondiert abhängig von dem verwendeten Kältemittel 1 mit einer bestimmten Siede- oder Verdunstungstemperatur. Diese Temperatur kann an die Steuerung 40 durch eine Bedieneinheit eingegeben werden, die durch den behandelnden Arzt bedient wird.

Die Steuerung 40 kann über das optionale Zulaufproportionalventil 32 den ersten Druck P₁, der über den Zulaufdruckfühler 31 bestimmbar ist, einstellen. Somit lässt sich beispielsweise über das Zulaufproportionalventil 32 die Menge des in die Kryosonde 10 eingebrachten Kältemittels 1 regulieren. Des Weiteren kann über das Einstellen der Druckdifferenz zwischen dem ersten Druck P₁ und dem zweiten Druck P₂ die Kühlleistung vorgegeben werden, die mittels des Joule-Thomson Effekts erzielt wird.

Die Fluidquelle 20 verfügt ebenfalls über ein optionales Fluidquellenventil 22, das ein manuelles Einstellen des Eingangsdrucks, mit dem das Kältemittel 1 in den Temperaturregler 30 eingebracht wird, ermöglicht.

Zusammenfassend wird noch einmal festgestellt, dass die Erfindung den zweiten Druck P₂ zur Reglung der Kühlleistung der Kryosonde 10 einstellt.

Der spezifische Siedepunkt eines Kältemittels 1 hängt bekanntlich von dem vorherrschenden Druck und der vorliegenden Temperatur ab. Bei einem vorgegebenen zweiten Druck P₂ siedet das Kältemittel 1 also nur bis zu einer spezifischen Temperatur. Soweit in dem Verdunstungsbereich 15 bereits eine niedrigere Temperatur vorliegt, siedet das Kältemittel 1 nicht. Dementsprechend wird dem System, begründet durch den Wechsel des Aggregatzustands, keine Wärme entzogen. Bei einer kontinuierlichen Zuführung von Kältemittel 1 bei konstantem zweiten Druck P₂ stellt sich also die entsprechende spezifische Temperatur in dem Verdunstungsbereich 15 ein.

### Bezugszeichen

- 1: Kältemittel
- 10: Kryosonde
- 11: Zulauf
- 12: Ablauf
- 14: Düse
- 15: Verdunstungsbereich
- 17: Sondenspitze
- 20: Fluidquelle
- 22: Fluidquellenventil
- 30: Temperaturregler
- 31: Zulaufdruckfühler
- 31': Ablaufdruckfühler
- 32: Zulaufproportionalventil
- 32': Ablaufproportionalventil
- 33: Durchflusssensor
- 40: Steuerung
- 42: Dateneingabevorrichtung
- P₁: Erster Druck
- P₂: Zweiter Druck

## Patentansprüche

1. Temperaturregler zur Reglung der Temperatur einer Kryosonde (10), welcher ein Kältemittel (1) mit einem ersten Druck (P₁) einem Verdunstungsbereich (15) derart zuführt, dass das Kältemittel (1) unter dem Vorliegen eines zweiten Drucks (P₂) zur Abkühlung eines Kühlabschnittes der Kryosonde (10) zumindest zeitweise verdampft, wobei eine Druckeinstelleinrichtung vorgesehen ist, welche zumindest den zweiten Druck (P₂) in dem Verdunstungsbereich (15) zur Reglung der Temperatur der Kryosonde (10) einstellt,
**dadurch gekennzeichnet, dass**
eine Strömungsmesseinrichtung (33), die zum Bestimmen einer Strömung ausgebildet ist die bei einem Abfluss des Kältemittels (1) aus dem Verdunstungsbereich (15) auftritt, und eine Kompensationseinrichtung, die zum Einbeziehen eines dem Abfluss entgegenstehenden Strömungswiderstands beim Messen und/oder Einstellen des zweiten Drucks (P₂) ausgebildet ist, vorgesehen sind.

2. Temperaturregler nach Anspruch 1,
**dadurch gekennzeichnet, dass**
die Druckeinstelleintichtung mindestens einen ersten Druckfühler (31') zum Messen des zweiten Druckes (P₂) und mindestens ein einstellbares Ventil (32') umfasst, die über eine Regeleinrichtung (40) zum Regeln des zweiten Drucks (P₂) verbunden sind.

3. Temperaturregler nach einem der vorhergehenden Ansprüche,
**gekennzeichnet durch**
mindestens einen Druckminderer zum Regeln des zweiten Drucks (P₂).

4. Temperaturregler nach einem der vorhergehenden Ansprüche,
**dadurch gekennzeichnet, dass**
die Strömungsmesseinrichtung (33) zum Bestimmen der Strömung einen zweiten Druckfühler (31) zum Erfassen des ersten Drucks (P₁) umfasst.

5. Temperaturregler nach einem der vorhergehenden Ansprüche,
**dadurch gekennzeichnet, dass**
die Kompensationseinrichtung eine Dateneingabevorrichtung (42) zur Eingabe von Strömungswiderstandsdaten der Kryosonde (10) umfasst.

6. Temperaturregler nach einem der vorhergehenden Ansprüche,
**dadurch gekennzeichnet, dass**
die Dateneingabevorrichtung (42) einen Datenspeicher umfasst, der Strömungswiderstandsdaten einer Vielzahl von Kryosonden (1) enthält.

7. Temperaturregler nach einem der Ansprüche 5 oder 6,
**dadurch gekennzeichnet, dass**
die Dateneingabevorrichtung (42) eine Geräteidentifikationseinheit zur Identifikation einer angeschlossenen Kryosonde (10) umfasst.

8. Temperaturregler nach einem der vorhergehenden Ansprüche,
**gekennzeichnet durch**
eine Expansionseinrichtung (14), die dem Verdunstungsbereich (15) zur mindestens teilweisen Kondensation des Kältemittels (1) vorgeschaltet ist.

9. Kryochirurgisches Gerät, umfassend eine Kryosonde (10) mit einem Sondenschaft und einen Temperaturregler (30) nach einem der vorhergehenden Ansprüche.

10. Kryochirurgisches Gerät nach Anspruch 9,
**dadurch gekennzeichnet, dass**
die Kryosonde (10) mindestens einen ersten Druckfühler umfasst, der zum Erfassen des zweiten Drucks (P₂) am oder nahe dem proximalen Ende des Sondenschafts angeordnet ist.

11. Verfahren zum Regeln der Temperatur einer Kryosonde (10),
wobei die Kryosonde (10) einen Verdampfungsbereich (15) zum Verdampfen eines Kältemittels (1) aufweist,
umfassend die Schritte;
- Bestimmen mindestens eines Drucks (P₂) des Kältemittels (1) im Verdampfungsbereich (15), umfassend das Bestimmen einer Strömung mittels einer Strömungsmesseinrichtung (33), die bei einem Abfluss des Költemittels (1) aus dem Verdunstungsbereich (15) auftritt, und das Einbeziehen eines den Abfluss endgegenstehenden Strömungwiderstands bein Messen und/oder Einstellen des zweiten Drucks (P₂) mittels einer dazu ausgestalteten Kompensationseinrichtungs;
- Einstellen des Drucks (P₂) auf einen vorbestimmten Wert, um die Temperatur der Kryosonde (10) zu regeln.

12. Verfahren nach Anspruch 11,
**dadurch gekennzeichnet, dass**
das Bestimmen des mindestens einen Drucks (P₂) mittels eines Druckfühlers (31') in der Kryosonde (10) an deren proximalem Ende oder bei der Ableitung des Kältemittels (1) aus der Kryosonde (10) erfolgt.

13. Verfahren nach einem der Ansprüche 11 oder 12,
**gekennzeichnet durch**
ein Drosseln des Kältemittels (1), um dieses zu verflüssigen.

## Claims

1. Temperature regulator for regulating the temperature of a cryoprobe (10), which temperature regulator delivers a refrigerant (1) at a first pressure (P₁) to an evaporation region (15) in such a way that the refrigerant (1) evaporates at least temporarily under the presence of a second pressure (P₂) in order to cool a cooling portion of the cryoprobe (10), wherein a pressure-setting means is provided that sets at least the second pressure (P₂) in the evaporation region (15) for regulating the temperature of the cryoprobe (10), **characterized in that** a flow-measuring means (33) is provided which is designed to determine a flow occurring when the refrigerant (1) flows out of the evaporation region (15), and a compensation means is provided which is designed to include a flow resistance, opposing the outflow, in the measuring and/or setting of the second pressure (P₂).

2. Temperature regulator according to Claim 1, **characterized in that** the pressure-setting means comprises at least a first pressure transducer (31'), for measuring the second pressure (P₂), and at least one adjustable valve (32') that are connected via a regulating means (40) for regulating the second pressure (P₂).

3. Temperature regulator according to one of the preceding claims, **characterized by** at least one pressure reducer for regulating the second pressure (P₂).

4. Temperature regulator according to one of the preceding claims, **characterized in that** the flow-measuring means (33) for determining the flow comprises a second pressure transducer (31) for detecting the first pressure (P₁).

5. Temperature regulator according to one of the preceding claims, **characterized in that** the compensation means comprises a data input device (42) for inputting flow resistance data of the cryoprobe (10).

6. Temperature regulator according to one of the preceding claims, **characterized in that** the data input device (42) comprises a data memory that contains flow resistance data of a large number of cryoprobes (10).

7. Temperature regulator according to either of Claims 5 and 6, **characterized in that** the data input device (42) comprises an apparatus identification unit for identifying a connected cryoprobe (10).

8. Temperature regulator according to one of the preceding claims, **characterized by** an expansion means (14) connected upstream of the evaporation region (15) for at least partially condensing the refrigerant (1).

9. Cryosurgical apparatus comprising a cryoprobe (10) with a probe shaft, and a temperature regulator (30) according to one of the preceding claims.

10. Cryosurgical apparatus according to Claim 9, **characterized in that** the cryoprobe (10) comprises at least a first pressure transducer which, for detecting the second pressure (P₂), is arranged at or near the proximal end of the probe shaft.

11. Method for regulating the temperature of a cryoprobe (10), wherein the cryoprobe (10) has an evaporation region (15) for evaporating a refrigerant (1), said method comprising the steps of:
- determination of at least one pressure (P₂) of the refrigerant (1) in the evaporation region (15), which involves
• determining, by means of a flow-measuring means (33), a flow that occurs when the refrigerant (1) flows out of the evaporation region (15), and
• including a flow resistance opposing the outflow in the measuring and/or setting of the second pressure (P₂), by means of a compensation means provided for this purpose;
- setting the pressure (P₂) to a predetermined value in order to regulate the temperature of the cryoprobe (10).

12. Method according to Claim 11, **characterized in that** the determination of the at least one pressure (P₂) by means of a pressure transducer (31') in the cryoprobe (10) takes place at the proximal end of the latter or upon removal of the refrigerant (1) from the cryoprobe (10).

13. Method according to either of Claims 11 and 12, **characterized by** restricting the refrigerant (1) in order to liquefy it.

## Revendications

1. Régulateur de température pour la régulation de la température d'une cryosonde (10), qui fournit un agent réfrigérant (1) avec une première pression (P₁) à une zone d'évaporation (15), d'une manière telle que l'agent réfrigérant (1) se vaporise au moins temporairement en présence d'une deuxième pression (P₂) pour le refroidissement d'une partie de refroidissement de la cryosonde (10), dans lequel il est prévu un dispositif de réglage de pression, qui règle au moins la deuxième pression (P₂) dans la zone d'évaporation (15) pour la régulation de la température de la cryosonde (10), **caractérisé en ce qu'**il est prévu un dispositif de mesure d'écoulement (33), qui est configuré pour déterminer un écoulement qui se produit lors d'une évacuation de l'agent réfrigérant (1) hors de la zone d'évaporation (15), et un dispositif de compensation, qui est configuré pour tenir compte d'une résistance à l'écoulement s'opposant à l'évacuation lors de la mesure et/ou du réglage de la deuxième pression (P₂).

2. Régulateur de température selon la revendication 1, **caractérisé en ce que** le dispositif de réglage de pression comprend au moins un premier capteur de pression (31') pour mesurer la deuxième pression (P₂) et au moins une vanne réglable (32'), qui sont reliés via un dispositif de régulation (40) pour la régulation de la deuxième pression (P₂).

3. Régulateur de température selon l'une quelconque des revendications précédentes, **caractérisé par** au moins un réducteur de pression pour la régulation de la deuxième pression (P₂).

4. Régulateur de température selon l'une quelconque des revendications précédentes, **caractérisé en ce que** le dispositif de mesure d'écoulement (33) destiné à la mesure de l'écoulement comprend un deuxième capteur de pression (31) pour déterminer la première pression (P₁).

5. Régulateur de température selon l'une quelconque des revendications précédentes, **caractérisé en ce que** le dispositif de compensation comprend un dispositif d'entrée de données (42) pour l'entrée de données de résistance à l'écoulement de la cryosonde (10).

6. Régulateur de température selon l'une quelconque des revendications précédentes, **caractérisé en ce que** le dispositif d'entrée de données (42) comprend une mémoire de données, qui contient des données de résistance à l'écoulement d'une multiplicité de cryosondes (10).

7. Régulateur de température selon l'une des revendications 5 ou 6, **caractérisé en ce que** le dispositif d'entrée de données (42) comprend une unité d'identification d'appareil pour l'identification d'une cryosonde raccordée (10).

8. Régulateur de température selon l'une quelconque des revendications précédentes, **caractérisé par** un dispositif de détente (14), qui est installé avant la zone d'évaporation (15) pour la condensation au moins partielle de l'agent réfrigérant (1).

9. Appareil cryochirurgical, comprenant une cryosonde (10) avec une tige de sonde et un régulateur de température (30) selon l'une quelconque des revendications précédentes.

10. Appareil cryochirurgical selon la revendication 9, **caractérisé en ce que** la cryosonde (10) comprend au moins un premier capteur de pression, qui est disposé à ou à proximité de l'extrémité proximale de la tige de sonde pour détecter la deuxième pression (P₂).

11. Procédé de régulation de la température d'une cryosonde (10), dans lequel la cryosonde (10) présente une zone d'évaporation (15) pour évaporer un agent réfrigérant (1), comprenant les étapes suivantes:
- déterminer au moins une pression (P₂) de l'agent réfrigérant (1) dans la zone d'évaporation (15), comprenant
- la détermination d'un écoulement au moyen d'un dispositif de mesure d'écoulement (33), qui se produit lors de l'évacuation de l'agent réfrigérant (1) hors de la zone d'évaporation (15), et
- la prise en compte d'une résistance à l'écoulement s'opposant à l'évacuation lors de la mesure et/ou du réglage de la deuxième pression (P₂) au moyen d'un dispositif de compensation conçu à cet effet;
- régler la pression (P₂) à une valeur prédéterminée, afin de réguler la température de la cryosonde (10).

12. Procédé selon la revendication 11, **caractérisé en ce que** la détermination de ladite au moins une pression (P₂) est effectuée au moyen d'un capteur de pression (31') dans la cryosonde (10) à l'extrémité proximale de celle-ci ou lors de l'évacuation de l'agent réfrigérant (1) hors de la cryosonde (10).

13. Procédé selon l'une des revendications 11 ou 12, **caractérisé par** un étranglement de l'agent réfrigérant (1) afin de le liquéfier.
